# EUROPEAN PATENT SPECIFICATION

(11) **EP 1 357 869 B1**
(45) Date of publication and mention of the grant of the patent: **13.04.2005**
(21) Application number: 02710641.8
(22) Date of filing: 07.02.2002
(51) Int. Cl.: A61F 7/12, A61M 25/10

(54) **BALOON CATHETHER FOR TREATMENT OF A MAMMALIAN DUCT OR CAVITY BY PRESSURE OR HEAT**
BALLONKATHETER ZUR BEHANDLUNG EINES DUKTUS ODER EINER HÖHLE IN SÄUGERN DURCH DRUCK ODER WÄRME
CATHETER A BALLONNET POUR LE TRAITEMENT D'UN CONDUIT OU D'UNE CAVITE DE MAMMIFERE PAR PRESSION OU CHALEUR

(30) Priority: 09.02.2001 SE 0100404
(43) Date of publication of application: 05.11.2003
(73) Proprietor: Wallsten Medical S.A., 1135 Denens (CH)
(72) Inventor: WALLSTEN, Hans, Ivar, CH-1135 Denens (CH)
(74) Representative: Lind, Urban
(86) International application number: PCT/SE2002/000212
(87) International publication number: WO 2002/064070

(56) References cited:
- WO-A1-99/07315
- WO-A1-99/08634
- US-A- 5 496 271

## Description

### Field of the invention

The present invention relates to balloon catheters for treatment of a mammalian duct or cavity using pressure and heat.

### Background of the invention

Catheters for the treatment of tissues using heat find many uses in medicine. Quite often the treatment resides in the use of relatively high temperatures to necrotise tissues, so called thermotherapy. Such treatment is frequently related to organs which are accessible only via narrow passages, the heat-releasing part of the catheter being arranged in the front or distal part of the catheter. The heat-releasing distal section as well as the catheter handle must have such a small diameter that the catheter can be introduced through a narrow passage at the same time as the passage must be protected from excessive heat.

In the treatment of so called menorraghia, i.e. abundant menstrual haemorrhage, using thermotherapy the mucous lining of the uterus cavity will be necrotised. A catheter is inserted transvaginally through the cervix canal which normally has a diameter of 4-6 mm. Even if the dimension of the cavity is considerably greater than the cervix canal the heat treatment can take place for example by using as a heat releasing distal section a high frequency cathode or microwave antenna which is moved back and forth in the cavity, the emitted heat being used to heat the tissue to be treated. The supply of power takes place by means of an electric lead via the catheter handle.

In the heat treatment of the uterus lining it is important that the narrow cervix canal will not be subjected to excess heat. In a similar manner benign prostatic hyperplasia, BPH, is treated using microwave heat and a small catheter containing electric lead and a distal antenna is inserted through penile urethra which has a diameter of 6-7 mm. The catheter is via an electric lead connected to an oscillator and a control unit. In the treatment microwaves are emitted from the antenna and are converted into heat in the prostate tissue.

In order to maintain a constant temperature the microwave antenna is cooled by circulating liquid, for example water. This also gives the advantage that the liquid forms a bridge between the antenna and the tissue which contributes to a homogeneous distribution of waves. Since the liquid will be heated it must be circulated and cooled. At the same time heat is moved from the tissue of the urinary tract, whereas the microwaves pass through the tissue and penetrate deeper into the tissue which is partially necrotised. In this manner the prostatic urethra is protected at least partly from burns, something which in certain cases is considered to accelerate the healing process.

The system with thermotherapy using microwaves is, however, complicated and expensive and is unsuited for use in doctors' office and is suitable only for use at major hospitals. Furthermore, the result is not convincing from a medical point of view.

An apparatus and a method for microwave treatment of BPH using also simultaneous dilation by means of a balloon is described in US patent 5,496,271 to Burton et al. From figures 7a-d and the corresponding passage of the description, column 15, line 31 to column 17, line 48, different embodiments for such cooling systems relating to direct cooling of the heat-releasing microwave antenna are described.

US patents 5,257,977 and 5,549,559 describe an apparatus for the treatment of inter alia BPH by means of a combination of dilation and heat. The apparatus is construed by a balloon catheter communicating with an external source of heat. The catheter is inserted through the urinary tract so that the balloon is positioned inside the prostate. A heated liquid is then circulated through the system under pressure so that the balloon will be expanded and will release heat under pressure for the treatment of the prostate.

According to available literature the treatment temperature is at most 60°C and the period of treatment about 45 minutes. In order to protect the rest of the urethra and the external sphincter from being heated the parts of the catheter excluding the balloon are thermally insulated by means of a plurality of sealed elongated closures, which contains trapped gas.

The skilled artisan knows that considerably higher temperature would be desirable for a good heat penetration in the prostate in order to obtain a deeper necrotisation of tissue. A higher temperature of for example 70-80°C, would also make a substantial shortening of the treatment time possible. This is important for several reasons, for example to reduce the degree of anaesthesia. As already mentioned it is important that only the affected prostatic tissue is treated and that the adjacent organs, for example the external sphincter, rectum and penile urethra, are not damaged in view of high temperatures.

'As mentioned, the diameter of the catheter should not exceed 6 and at most 7 mm in order to be capable of insertion through the urinary tract. Accordingly, there are strict limitations concerning thickness of insulation, since the central part of the catheter must have accommodation for circulating liquid, drainage tube for urine, etc. Experiments have shown that the surface temperature of the insulation in a device similar to that described in US patent 5,257,977 will be about 40-41°C at a temperature of a circulated liquid of 60°C.

43°C is considered to be the temperature which as a maximum can be allowed to avoid pain and burn damages. Another limitation with the above method is that 9 different catheters are needed to treat prostate lengths of between 2 and 6 cm.

### Objects and summary of the invention

The present invention is defined in claim 1. It has for an object to provide a balloon catheter for heat treatment under pressure surrounding tissue of mammalian duct or cavity while avoiding excessive heating of adjacent organs or sites.

Another object of the invention is to provide a balloon catheter, wherein such excessive heating is avoided by the use of a combination of a heat-insulating medium and a liquid cooling medium, said media being substantially co-extensive within the area to be protected from excessive heating.

Yet another object of the invention is to provide a catheter allowing flow of cooling medium past said co-extensive hest-insulating medium on the outside thereof.

A further object of the invention is to provide a balloon catheter enabling variation of the balloon length to match treatment sites of various extensions.

Still another object of the invention is to provide a balloon catheter useful for heat treatment under pressure of the prostata.

A further object of the invention is to provide a balloon catheter carrying a radially expansible stent encompassing the balloon and capable of being implanted after radial expansion and heat treatment upon withdrawal of the catheter from the site of treatment.

Still another object of the invention is to provide a balloon catheter having a stent made of a thermoplastic biodegradable polymer deformable using heat at a temperature above body temperature.

The above and other objects of the invention will be readily apparent from the following description involving a summarising part of the invention a description of specific embodiments thereof.

The balloon catheter according to the invention for exerting internal pressure on surrounding tissue of a mammalian duct or cavity comprises an elongate distal section and a flexible and expandable balloon accommodating said distal section which, together with said balloon is intended for insertion into said duct or cavity. Furthermore, means are provided for the supply of a pressure medium for expansion of said balloon and heating means for heating said medium. The catheter further comprises an intermediate section and a proximal section for operating the device, said distal and intermediate sections containing a central tube, whose distal part is provided with at least one outlet for said medium within said balloon, and whose intermediate part is surrounded by an axially extending tube, at whose distal end the proximal end of said balloon is attached, the distal end of said balloon being attached to said central tube. By the expression "at whose distal end" is meant that the proximal end of the balloon is attached either to the central tube or to the axially extending tube.

The balloon catheter according to the invention is characterised by an intermediate tube concentrically positioned within said axially extending tube and surrounding said central tube so as to form an annular space between said central tube and said intermediate tube containing a heat-insulating medium and a second annular space between said intermediate tube and said axially extending tube, the second annular space being divided into at least two separate axially extending compartments by at least two opposite radial partitions, said compartments being in fluid communication at the distal ends thereof, and one compartment having a proximal inlet for a cooling medium and another compartment having a proximal outlet for said cooling medium.

It is preferred to arrange for another tube which surrounds the central tube and forms a distally and proximally open annular space between said another tube and the central tube, said annular space allowing return and recirculation of a pressure medium introduced into the balloon.

Said heating means can be positioned within the balloon, in the intermediate section, or externally.

Circulation means are suitably provided which generate circulation of said medium within or through the balloon.

The heating means is preferably selected from electric resistance elements, PTC resistors, laser energy or micro wave energy emitting means, and external heat exchangers.

In a particularly preferred embodiment of the invention the balloon catheter is designed so that the axially extending tube together with said intermediate tube forms a unit which is axially displaceable in relation to the distal end of the balloon, and so that the proximal end of said balloon is attached to the distal end of said unit so as to confer adjustability of the axial length of the balloon. Such embodiment further comprises locking means for a desired balloon length as axially adjusted.

As an alternative for providing balloon length adjustment the central tube is composed of at least two telescopic concentric partially overlapping tubes, one of which protrudes distally and carries the distal end of the balloon, and the other one of which carries the proximal end of the balloon whereby axial relative displacement of said tubes enables balloon length adjustment. Also in this embodiment the catheter comprises locking means for maintaining a desired balloon length.

Further, in the balloon catheter of this invention it is preferred that the radial extension of said axially extending tube, said intermediate tube, and said central tube including the annular spaces therebetween occupy from about 15% to about 40% of the catheter radius.

According to a preferred embodiment the radial partitions ending short of the distal end of the axially extending tube are formed by axially extending interior ridges defined by corresponding exterior grooves.

According to an alternative embodiment the intermediate tube may be provided with axially extending exterior grooves accommodating flexible threads forming the radial partitions. To establish fluid communication between the compartments the partitions end short of the distal end of the intermediate tube.

For the treatment of the prostate the balloon catheter according to the invention is preferably provided with a heat insulation at the proximal end of the balloon for the protection of the sensitive area adjacent to sphincter. In this connection it is particularly preferred that said heat insulation is extended towards the rectum area to protect the part of the prostate cavity facing the thin rectum wall.

In another preferred embodiment of the invention the balloon catheter is provided with a radially expansible stent encompassing the balloon. Such stent is deformable to expand synchronously with the radial expansion of the balloon when inserted, thereby being retained expanded in the treatment site when the catheter is drawn from the duct or cavity.

It is preferred that such stent is made of a thermoplastic biodegradable polymer which is deformable with heating by having a softening point above body temperature. The polymer is preferably selected from PCA, PLLA and PLGA and co-polymers thereof.

An alternative material for such stent is constituted by a so called memory metal having a certain transition temperature above which the material and thereby the stent returns to a preprogrammed expanded state.

The catheter of the present invention allows to practice a method for a treatment of a mammalian duct or cavity site by heating surrounding tissue thereof, and said site is reached via heat-sensitive area. Such method comprises the steps:
a) passing a heating medium through said area to said site; and
b) radially surrounding said medium when passing said area with a heat insulating layer which in turn is radially surrounded by a heat-diverting medium, thereby protecting said area from excessive heating.

It is preferred that the heat-diverting medium is a flowing liquid, such as water or an aqueous solution.

For prostate treatment the heating is preferably formed at a temperature of from about 60 to about the boiling point of water, and the temperature at said heat-sensitive area is kept at a value not causing tissual damage, such as lower than about 42 to 45°C.

An alternative method which may be practiced with the catheter according to the invention comprises the steps:
a) applying onto a balloon a radially expansible stent capable of radial deformation;
b) distending said balloon by introducing a pressurised fluid therein to exert pressure on said tissue to widen to a predetermined degree said site and to expand the stent by using a balloon of predetermined limited radial expansion thereby expanding the stent; and
c) removing fluid from said balloon and withdrawing same in a collapsed state from said site leaving the stent behind in an expanded state.

The treatment using pressure and heating is suitably maintained for a period of time to achieve a therapeutic effect in the tissue to be treated.

In a method using the catheter according to the invention wherein the stent is made of a thermoplastic biodegradable polymer having a softening point above body temperature, the method suitably comprises the steps:
a) inserting said balloon carrying said stent into a prostatic urethra;
b) expanding said balloon by introducing a pressurised fluid and supplying heat to soften and expand the stent and to widen said urethra to a predetermined degree;
c) maintaining pressure and heating for a period of time and at a temperature resulting in partial necrosis of prostatic tissue;
d) interrupting heating for lowering the temperature to fortify the stent; and
e) releasing the pressure to deflate the balloon and withdrawing same while leaving the stent behind in an expanded state.

As a further refinement of the method a further step preceding step d) may be added residing in partial deflation of the balloon to a desired final degree of expansion of the stent.

In the instant disclosure the expressions "distal" and "proximal" are used with the meaning "front" and "rear", respectively, i.e. related to the operator of the catheter.

### Detailed description of the invention

The invention will in the following be further described by exemplifying embodiments which, however, must not be construed to restrict the scope of protection except as defined in the appended claims. These embodiments are described with reference to the appended drawings, wherein:
Fig. 1 is a simplified diagrammatic illustration of a previously described balloon catheter described in a pending Swedish patent application;
Fig. 2 is an enlarged diagrammatic illustration in section of part of the device according to the present invention;
Fig. 3 is a section along line A-A of the embodiment according to Fig. 2;
Fig. 4 is a perspective view of a detail of the embodiment shown in Fig. 2;
Fig. 5 is a simplified diagrammatic illustration by a section through the catheter's two outer tubes showing the partition arrangement;
Fig. 6 is an alternative arrangement showing another way of providing partitions;
Fig. 7 is a simplified diagrammatic illustration of a section of a detail of an alternative cooling arrangement of the catheter;
Fig. 8 is a perspective view of a detail shown in Fig. 7;
Fig. 9 is a simplified diagrammatic view of the distal end of a catheter embodiment provided with proximal balloon insulation;
Fig. 10 is an end view of the embodiment shown in Fig. 9;
Fig. 11 is a diagrammatic view in section of a specific insulation design;
Fig. 12 is a simplified illustration of the distal end of a balloon catheter according to the invention enabling an alternative arrangement for adjusting the axial extension of the balloon; and
Fig. 13 shows an enlarged view in section a detail as seen along line A-A in Fig. 12.

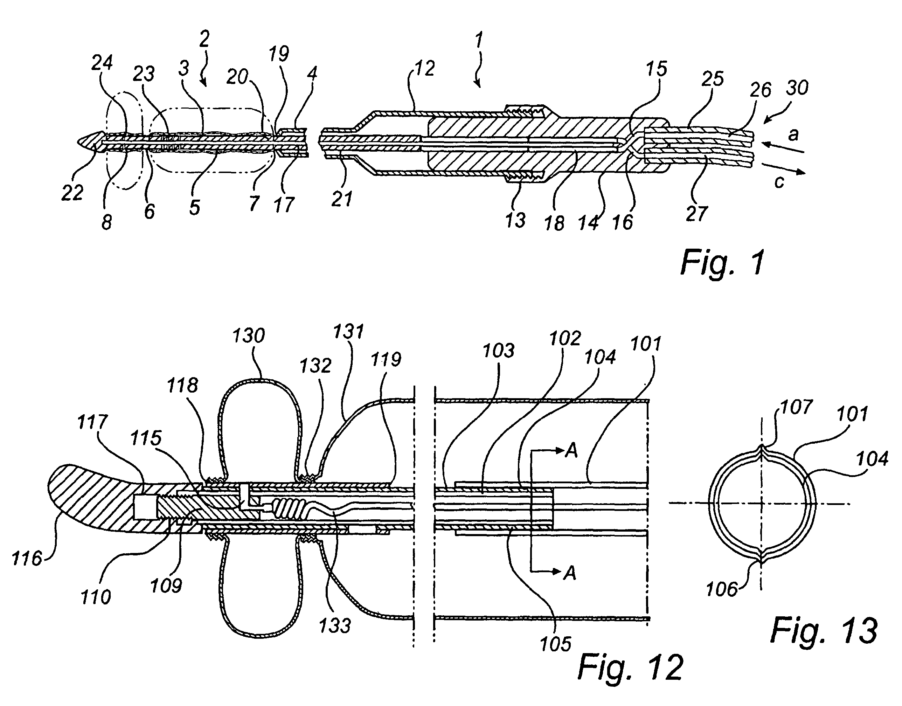

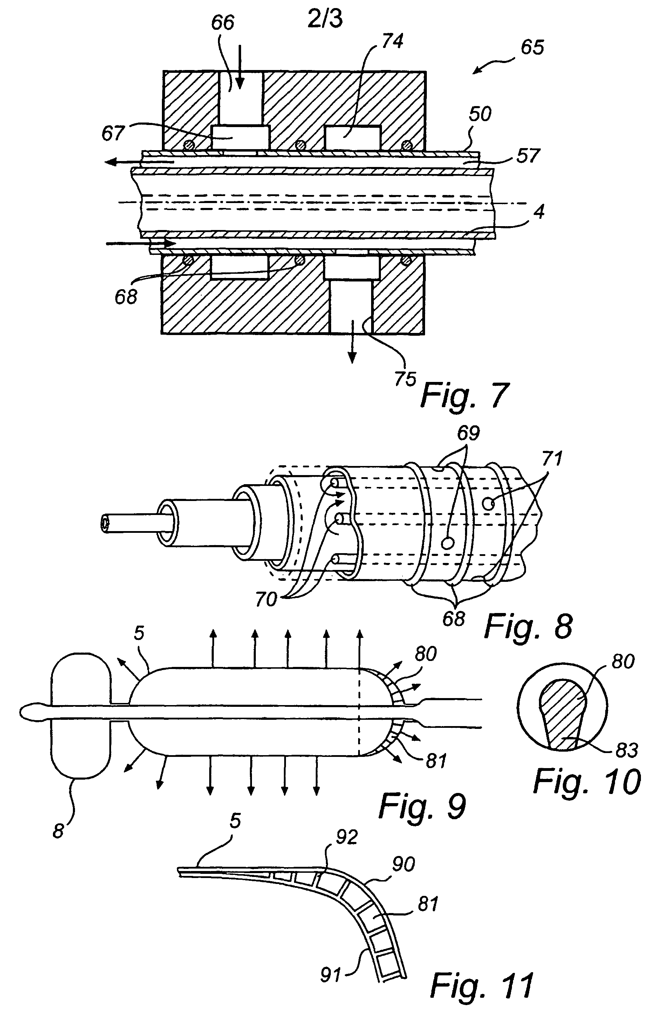

In the previously described device for the treatment of inter alia BPH, one embodiment of this device is diagrammatically shown in a Figure 1. The catheter shown in Figure 1 and generally designated 1 is useful for the treatment of benign prostate hyperplasia (BPH) using pressure and heat. As seen to the left in Figure 1 there is shown the distal end 2 of the catheter intended for insertion from the outside through the urethra and which comprises two concentric tubes 3,4 which are axially displaceable relative each other, tube 4 partly enclosing tube 3. An elastic, inflatable balloon 5 for the treatment with pressure and heat is at its distal end attached to tube 3 at 6 and at its proximal end attached to tubes 4 and 17 at 7. At the outermost distal end of catheter 1 a second, inflatable balloon 8 is arranged for positioning of the catheter during treatment so that the active part of balloon 5 will be correctly positioned between bladder neck and the external sphincter.

Balloons 5 and 8 are in Figure 1 shown by full lines in deflated condition and by dashed lines in expanded position.

Catheter 1 also includes an intermediate section 11 containing an enlargement 12 of tube 4 and a locking nut 13 together forming a handle. The enlargement 12 and the locking nut 13 surround a body 14 which is interiorally connected to tube 3. Accordingly, the length of the elastic balloon 5 can be varied by displacement of the handle. The length of the balloon can be read on a scale on body 14 and the desired length can be maintained by turning the locking nut 13.

Catheter 1 has a proximal part 30 shown significantly shortened and comprising a flexible tube 25 for connection to a control unit (not shown) and conduits 26,27 for the supply of medium for the expansion of balloons 5 and 8 and for the circulation of heating medium from an external heat source through balloon 5.

The body 14 contains passages 15 and 16 connected to. a flexible multilumen tube 25 containing an inlet lumen 26 and an outlet lumen 27 for the introduction of a heating medium and discharge of the medium, respectively (arrows a and c). Concentric to the tube 3 and the axially displaceable tube 4 there is arranged an intermediate tube 17 extending from the distal end of tube 4 and proximally into a bore 18 provided in the body 14. At the distal end thereof the intermediate tube 17 is sealingly attached to an inward flange 19 on the tube 4. In this manner tubes 4 and 17 can be axially displaced as a unit for the adaptation of the balloon length as described above. By the concentric arrangement of the intermediate tube 17 around tube 3 there is provided an outlet annular passage gap opening 20 providing connection inside the body 14 with the outlet passage 16. Between the intermediate tube 17 and the surrounding axially displaceable tube 4 an insulating annular space 21 is formed acting as a heat insulation to prevent excessive heating of the urethra.

Openings 23 in tube 3 provide connection between the inlet lumen 26, the passage 15, the interior of the tube 3 and the interior of the balloon 5. At the distal section of the tube 3 a distal body 22 is arranged wherein tube 3 is inserted. Radial openings 24 in body 22 provide connection with a fine tube (not shown) positioned in the inlet lumen 26, extending along the passage 15, inside the tube 3 and into the interior of the balloon 8.

As mentioned above tube 25 is intended for connection to a control unit (not shown) from which a heat-carrying medium, for example a liquid, can be introduced into the catheter. During treatment the medium is heated in an externally arranged heat exchanger to the desired temperature and is circulated by means of an external pump in a circuit comprising pump, heat exchanger, control unit for inter alia control of pressure, temperature, amount of liquid, etc.

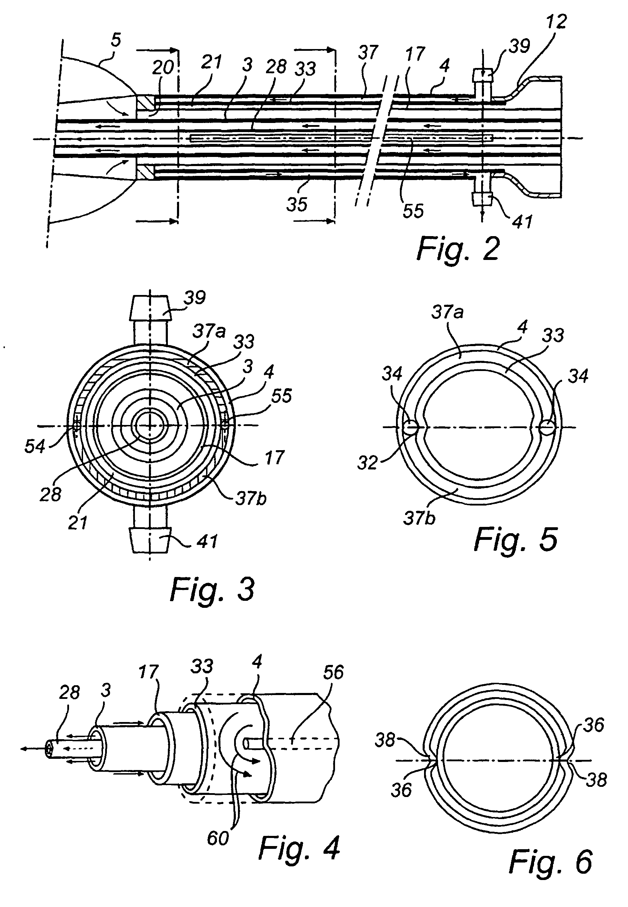

Figure 2 shows a detail of an embodiment of the catheter according to the invention for heat- and pressure-treatment of an organ, for example the prostate. In this embodiment the balloon catheter operates according to a similar principal as described in relation to the catheter shown in Figure 1. Figure 2 only shows the section of the catheter comprising the catheter handle corresponding to the section between the balloon attached at 7 and the enlargement of the intermediate section 12. It is this section of the catheter which during the treatment using heat and pressure is positioned in the heat-sensitive penile urethra.

The reference numerals used in Figure 1 are correspondingly used in relation to Figure 2. Thus, only the proximal part of the balloon 5 is shown in Figure 2, the central tube 3 is intended for the supply of hot pressure medium, and tube 17 enables removal of the circulating hot medium through outlet 20. A concentric tube 33 inside tube 4 defines a space 21 for heat insulation. A fine supply tube 28 is positioned inside tube 3 and enables supply of pressure medium for expansion of the positioning balloon 8 shown in Figure 1.

In the embodiment of the invention shown in Figure 2 three concentric tubes 4, 33, 17 are sealingly attached at their proximal ends to a flange 19, whereas tube 33 at the proximal end is sealingly attached to the tube 12. Between tubes 4 and 33 an annular space 37 is formed having a proximal inlet 39 for cooling liquid and an oppositely placed outlet 41.

In Figure 3 there is shown a section A-A through the catheter detail shown in Figure 2. In the annular space 37 between tubes 4 and 33 two axially extending and oppositely positioned sealing partitions 54 and 55 are arranged which divide space 37 into two parts, one upper part 37a and one lower part 37b. One partition 55 is shown with dashed lines in Figure 2. Proximally the two partitions extend from a proximal position between inlet 39 and outlet 41, whereas at the distal end the partitions extend short of the flange 19, thereby forming connection between the two parts 37a, 37b of the annular space 37.

When a liquid, for example water, is supplied to inlet 39 the liquid will form a film filling the upper half 37a and flowing axially up to the distal end of the upper part 37a to turn downwardly and flow back in the lower part 37b and out through outlet 41.

As a safety measure tube 4 can be preferably made of a plastic material having incorporated therein a colour-changing material. Such material is designated to change colour at a desired temperature, said change starting at for example about 38°C and being completed at about 41°C. The material is composed of a bright-coloured pigment encapsulated in another less coloured pigment which, at the desired temperature, melts and becomes transparent thereby making the bright-coloured pigment visible. An example of such material is Chromazone.

Figure 4 shows in a perspective view a detail of the distal part of the catheter section shown in Figure 2 illustrating the concentric tubes 28,3,17,33 and 4 and a partition 56. Arrows 60 show how the flowing cooling liquid turns around the distal end of partition 56 at the distal ends of tubes 4,33.

As previously mentioned it is important, in for example the treatment of prostatic disorders, for example BPH, to make only the prostate tissue subject to treatment by a combination of pressure and heat. It has also been found that the highest temperature which can be used in treatment with a circulating liquid and using only heat-insulation of the catheter handle is about 60°C.

Allowing a catheter diameter of at most 7 mm the thickness of the heat-insulation can hardly exceed 1,5 mm, since there must be space for the necessary arrangement for circulation, pressure control etc, and such heat-insulation is not sufficient when using treatment temperatures above 60°C. For temperatures between for example 75°C and 90°C a surface temperature of about 40°C for avoiding heating damages would require a thickness of the heat-insulation corresponding to a catheter diameter of 9-10 mm. Such catheter diameter cannot be used for the treatment of BPH since it would not allow excess through the penile urethra.

It has now surprisingly been found that it is possible by using a catheter design as described in connection with Figures 2 to 4 to produce a catheter having a diameter as small as about 4.5 to 5 mm, the intermediate section of which when circulating a hot liquid of up to 90°C and more can have an exterior temperature of below 40°C without using complicated cooling devices but merely water of room temperature at a very low flow rate.

To this end the catheter according to the invention as described contains five concentric tubes, of which the three inner tubes 28,3,17 two are used for the circulation of hot heating medium and one for inflating the treatment and positioning balloons. The function of the outer tubes 4 and 33 is to provide an exterior temperature on tube 4 not exceeding for example about 40°C.

Practical experiments have surprisingly shown that this can be provided with a very thin annular insulation 21 of air and a very thin liquid flowing annular film 37. As an example there can be mentioned that experiments have shown that an air gap 21 of 0.15 mm and a water film 37 of 0.15 mm were fully sufficient to keep the temperature on the exterior surface of the catheter tube 4 at 32-35°C at such a minute flow of water of 25°C as 15 to 20 ml/min equivalent to about 2 drops/sec. The quantity of circulating hot water for the heat treatment was in this case about 100 ml/min. at temperatures between 90°C and 65°C. The latter temperature range is fully sufficient to provide a therapeutic effect using a balloon catheter of the described design. It is also surprising that the exterior temperature on tube 4 seems to be substantially independent of the temperature of the circulating hot water.

A very simple and cheap device can be used for controlling that the necessary quantity of water flows through the annular space 37. One way is to use the infusion systems used by hospitals for intravenous supply of liquid where the liquid supply can be adjusted and an alarm signal alerted into function if the supply will be interrupted.

As mentioned above the quantities of liquid are very minute - of the order of about 2 drops/sec., and therefore the liquid need not be circulated but can be collected in a container. The total quantity of liquid for half an hour of treatment is thus only about 0.5 L.

In the above described embodiment the catheter handle contains five concentrically arranged tubes. It is an advantage if these are thin so that the catheter handle obtains the smallest possible diameter yet leaving the necessary space for circulation of liquid and other functions necessary for the treatment.

Catheters having the design shown in Figure 2 have been made with an outer diameter of 5 mm, which is very advantageous for trans-urethral insertion. The tubes can be made of thin stainless steel having a wall thickness of between 0.15 and 0.25 mm. The outer diameter of tube 17 in Figure 3 containing all necessary functions, such as passages for heat water circulation, is in this case 3.4 mm. Accordingly, the difference in diameter is 5-3.4 mm = 1.6 mm. Thus, a total of wall thickness of about 0.8 mm is taken by the outer sleeve comprising the thickness of tubes 4 and 33 and space 37 for a thin cold water film and space 21 for a thin annular of air. Totally, 1.6 mm have been taken from 5 mm equal to 30% to reduce the surface temperature of tube 17 from for example 90°C to 40°C and with very low heat losses. With a catheter diameter of at most 7 mm the corresponding value would have been about 20%.

As previously mentioned it is important in the heat treatment of the prostate to avoid damages to adjacent organs, such as the sphincter and the rectum. On the other hand it is often quite important to heat treat the prostate tissue also close to the sphincter.

In the previously disclosed device according to a pending Swedish patent application an arrangement is described whereby the treatment balloon 5 can be adjusted in axial extension so that when necessary treatment close to the sphincter can be made. This can initiate a situation where heat spreads axially from the proximal attachment of balloon 5 and along a length of outer tube 4 passing inside the sphincter. By using the design according to the present invention this risk will be eliminated in view of the fact that heat adjacent to flange 19 will be removed from the outer tube 4.

Figures 5 and 6 show two different designs relating to the partitions for dividing annular space 37 into an upper part 37a and a lower part 37b. The embodiment according to Figure 5 is constituted by arranging inwardly directed grooves 32 on tube 33, said grooves accommodating elements or threads 34 acting as partitions and extending axially along tube 33. Such grooves can be made on tube 33 without changing the circular interior configuration of the tube.

Figure 6 shows an alternative arrangement, according to which the outer tube 4 is provided with inwardly extending axial ridges 36 and corresponding outward grooves 38, said ridges forming the desired partitions.

According to yet another alternative it is possible to simultaneously co-extrude tubes 4,33 together with the radial partitions to form an integral multi-lumen tube construction. This is particularly the case when using as a material a thermoplastic polymer.

The invention is not restricted to the design for the formation of a thin, flowing film of water as described in connection with Figures 2 to 4, and an alternative design is described below.

Figures 7 and 8 show a detail of another embodiment of the invention, according to which the flowing liquid film is divided into several parts flows. Figure 7 shows a liquid distributor 65 having an inlet 66 for water leading to a chamber 67 which liquid-tight by means of O-rings 68 fully encloses a tube 50. From chamber 67 a number of inlet openings 69 lead to an annular space 51 formed between tubes 4,50. In this case space 51 is divided up by a number of axially extending sealing partitions 70, as shown in Figure 8 also showing the O-rings 68. Accordingly, water will pass through inlet openings 69, will be guided axially between the sealing partitions 70 towards the distal part of the catheter in the same manner as shown in connection with Figure 4, the water flow turns as shown by arrows 72 and flows axially back into the proximal part of the catheter handle and will be discharged through outlet 75 via outlet openings 71 and chamber 74.

Yet another embodiment of the invention is shown in Figure 9 illustrating the distal part of the balloon catheter of similar type as that described in Figures 1 and 2. The balloon catheter according to this embodiment is particularly suitable for heat treatment of the prostate and includes in this case a positioning balloon 8, an elastic, inflatable balloon 5 for the heat treatment of the prostate and a catheter tube 4, wherein the proximal part of balloon 5 is attached at 7.

Balloon 5 which for example is constituted by a preformed flexible material, is double-walled, at least at the proximal end 80 of the balloon to form a space 81 restricting the heat-release from the heating medium within the balloon at the proximal end of balloon 5.

Space 81 can be filled with air, but since the balloon material has a certain gas penetrability and a certain pressure exists in the space when the balloon is inflated causing risk for collapse of space 81, it can alternatively be filled with a material of good heat-insulating capacity. In order to facilitate the movability of the balloon material during expansion the insulating material can consist of a balanced quantity of porous spheres or an elastic, porous weave.

By this embodiment of the invention the heat convection towards the sphincter positioned adjacent to the proximal end of the balloon will be reduced as illustrated by arrows 82 of different length in Figure 9.

Figure 10 shows the insulation 80 as seen distally. In this case the insulation has been extended at 83 to restrict the heat convection in direction towards the part of the prostate cavity which is facing the rectum wall, said wall being thin and sensitive to influence by heat.

Figure 11 shows another embodiment of the heat insulation. The double-walled balloon 5 has an exterior wall 90 and an interior wall 91, the latter being provided with a number of small protrusions 92. In this manner an air-filled insulating space 81 is formed when the balloon is inflated. The interior part of the balloon can be easily manufactured by injection moulding of for example a silicon material. Suitable height of the protruding parts is for example about 1 mm and a distance of about 1 mm between each part.

Figure 12 shows an alternative arrangement for adjusting the axial length of the balloon to match treatment sites of different sizes. The device shown in Figure 12, illustrating the distal part of the balloon catheter only, contains two telescopically arranged tubes, a central tube 101 and a telescopic tube 102 within the central tube 101 and an intermediate tube 103. All tubes fit tightly within each other and are axially displaceable relative to each other. A stop ring 104 is attached onto and at the proximal end of the telescopic tube 102 and is constituted by a short tube having the same size as the intermediate tube 103. Between tube 103 and the stop ring 104 a locking ring 105 of an elastic material is arranged. The central tube 101 has two extensions 106 and 107 extending axially. In a corresponding manner there are two extensions in stop ring 104 which have for an effect that the telescopic tube 102 having the stop ring 104 and the intermediate tube 103 can be axially displaced in central tube 101 but not rotated. If on the other hand the intermediate tube 103 is axially displaced in relation to the telescopic tube 102 and the stop ring 104 so that the elastic ring 105 will be expanded, then the telescopic tube 102 will be locked in relation to the central tube 101.

In the distal part of the telescopic tube 102 an elongate body 109 has been firmly inserted. Body 109 is provided with an exterior thread 110 and contains a canal or passage 115.

The distal part 116 of the catheter can be permanently bent upwardly and contains a bore 117 having an interior thread co-operating with thread 110 in body 109.

A sleeve 118 is attached to and surrounds the distal part of intermediate tube 103. The proximal part 119 of this sleeve 118 acts as a stopper against the distal part of the central tube 101 when the telescopic tube is axially pushed into central tube 101.

The distal part 116 of the catheter is threaded onto body 109 so that its proximal part rests against sleeve 118 and the distal part of intermediate tube 103 at the same time as the proximal part of the intermediate tube 103 without force rests against ring 105 which in turn rests against stop ring 104. In this position telescopic tube 102 can be axially displaced forwardly or rearwardly in central tube 101.

A positioning balloon 130 and treatment balloon 131 are shown in Figure 12, and the distal part 132 of the treatment balloon 131 as well as the positioning balloon 130 are attached to sleeve 118. The proximal part of treatment balloon 131 is attached to the central tube 101 (not shown in the Figure).

The axial length of the treatment balloon 131 can be adjusted before treatment by displacing telescopic tube 102 in relation to central tube 101 until the distance between the proximal attachment of the treatment balloon 131 onto central tube 101 and the distal attachment to telescopic tube 132 corresponds to the desired length. Locking of this position takes place by rotating the distal part 116 to engage the intermediate tube 103 against locking ring 105 so as to result in expansion thereof.

Figure 12 also shows a flexible capillary tube 133 through which air can be injected to expand the positioning balloon 130. By designing the distal part of the capillary tube 133 as a coil the capillary tube can move along with extension or shortening of the telescopic arrangement.

The alternative arrangement for providing adjustability of the axial length of the treatment balloon as shown in Figures 12 and 13 gives an added advantage in relation to the design according to Figure 1. By moving the system for adjustment of axial length of the treatment balloon from the intermediate or handle part of the catheter to the distal part thereof greater freedom will be obtained in regard to the design of the intermediate part of the catheter. Accordingly, this part can be made flexible or can be given a bent shape for better accommodation to a particular treatment site, such as the prostate.

It is to be noted that many variations of the invention as described are conceivable and within the skill of the artisan, and the invention is to be limited solely by the scope of the appended claims.

## Claims

1. Balloon catheter for exerting internal pressure on surrounding tissue of a mammalian duct or cavity in a section thereof, comprising an elongate distal section and a flexible and expandable balloon accommodating said distal section which, together with said balloon is intended for insertion into said duct or cavity section, further comprising means for the supply of a pressure medium for expansion of said balloon and heating means for heating said medium, the catheter further comprising an intermediate section and a proximal section for operating the device, said distal and intermediate sections containing a central tube, whose distal part is provided with at least one outlet for said medium within said balloon, and whose intermediate part is surrounded by an axially extending tube, at whose distal end the proximal end of said balloon is attached, the distal end of said balloon being attached to said central tube, **characterized by** an intermediate tube concentrically positioned within said axially extending tube and surrounding said central tube so as to form an annular space between said central tube and said intermediate tube containing a heat-insulating medium and a second annular space between said intermediate tube and said axially extending tube, the second annular space being divided into at least two separate axially extending compartments by at least two opposite radial partitions, said compartments being in fluid communication at the distal ends thereof, and one compartment having a proximal inlet for a cooling medium and another compartment having a proximal outlet for said cooling medium.

2. Balloon catheter according to claim 1, **characterized by** another tube surrounding said central tube so as to form a distally and proximally open annular space between said another tube and said central tube allowing return and recirculation of pressure medium introduced into said balloon.

3. Balloon catheter according to claim 1 or 2, wherein said heating means is positioned within said balloon.

4. Balloon catheter according to claim 1 or 2, comprising circulation means generating circulation of said medium within or through said balloon.

5. Balloon catheter according to claim 2, wherein said heating means is positioned in said intermediate section.

6. Balloon catheter according to claim 2, wherein said heating means is positioned externally of said sections.

7. Balloon catheter according to any preceding claim, wherein said heating means is selected from electric resistance elements, PTC resistors, laser energy emitting means, and external heat exchangers.

8. Balloon catheter according to any preceding claim, wherein the axially extending tube together with said intermediate tube forms a unit which is axially displaceable in relation to the distal end of said balloon, and wherein the proximal end of said balloon is attached to the distal end of said unit so as to confer adjustability of the axial length of the balloon, the catheter comprising locking means for maintaining a desired balloon length.

9. Balloon catheter according to any one of claims 1 to 7, wherein said central tube is composed of at least two telescopic and concentric partially overlapping tubes, one of which protrudes distally and carries the distal end of the balloon, and the other one of which carries the proximal end of the balloon whereby axial relative displacement of said tubes enables balloon length adjustment, the catheter comprising locking means for maintaining a desired balloon length.

10. Balloon catheter according to any preceding claim, wherein the radial extension of said axially extending tube, said intermediate tube, and said central tube including the annular spaces therebetween occupy from about 15% to about 40% of the catheter radius.

11. Balloon catheter according to any preceding claim, wherein said axially extending tube is provided with axially extending interior ridges defined by corresponding exterior grooves, said ridges forming said radial partitions ending short of the distal end of said axially extending tube, thereby establishing said fluid communication between said compartments.

12. Balloon catheter according to any one of claims 1 to 10, wherein said intermediate tube is provided with axially extending exterior grooves accommodating flexible threads forming said radial partitions, said partitions ending short of the distal end of said intermediate tube thereby establishing said fluid communication between said compartments.

13. Balloon catheter according to any preceding claim for the treatment of the prostate, wherein said balloon at the proximal end thereof is provided with a heat insulation for the protection of the sensitive area adjacent to sphincter.

14. Balloon catheter according to claim 13, wherein said heat insulation is extended toward the rectum area to protect the part of the prostate cavity facing the thin rectum wall.

15. Balloon catheter according to any preceding claim, **characterized by** a radially expansible stent encompassing said balloon, said stent being deformable to expand synchronously with the radial expansion of the balloon when inserted in said duct or cavity, thereby being retained expanded therein upon withdrawal of the catheter from said duct or cavity.

16. Balloon catheter according to claim 15, wherein said stent is made of a thermoplastic biodegradable polymer which is deformable with heating by having a softening point above body temperature.

17. Balloon catheter according to claim 16, wherein said polymer is selected from PCA, PLLA and PLGA and co-polymers thereof.

18. Balloon catheter according any one of claims 15 to 17, wherein said stent has been made subject to heat forming at a temperature near melting temperature to give a desired stent configuration as implanted, and then formed to a desired configuration for insertion.

## Patentansprüche

1. Ballonkatheter zum Ausüben von innerem Druck auf umliegendes Gewebe eines Gangs oder einer Höhle eines Säugers in einem Abschnitt des Gangs oder der Höhle mit:
einem länglichen distalen Abschnitt und einem flexiblen und dehnbaren Ballon, der den distalen Abschnitt aufnimmt, der zusammen mit dem Ballon zum Einführen in den Gangoder Höhlenabschnitt vorgesehen ist;
Mitteln zum Zuführen eines Druckmediums zum Ausdehnen des Ballons und Heizmitteln zum Aufheizen des Mediums und
einem Zwischenabschnitt und einem proximalen Abschnitt zum Betreiben der Vorrichtung, wobei der distale und der Zwischenabschnitt einen Mittelschlauch enthalten, dessen distaler Teil mit mindestens einem Auslass für das Medium in dem Ballon versehen ist und dessen Zwischenteil von einem axial verlaufenden Schlauch umgeben ist, an dessen distalem Ende das proximale Ende des Ballons befestigt ist, wobei das distale Ende des Ballons an dem Mittelschlauch befestigt ist,
**gekennzeichnet durch** einen Zwischenschlauch, der konzentrisch in dem axial verlaufenden Schlauch angeordnet ist und den Mittelschlauch umgibt, sodass ein ringförmiger Raum zwischen dem Mittelschlauch und dem Zwischenschlauch, der ein wärmedämmendes Medium enthält, und ein zweiter ringförmiger Raum zwischen dem Zwischenschlauch und dem axial verlaufenden Schlauch entstehen, wobei der zweite ringförmige Raum von mindestens zwei gegenüberliegenden radialen Trennwänden in mindestens zwei getrennte axial verlaufende Kammern unterteilt wird, wobei die Kammern an ihren distalen Enden flüssigkeitsgekoppelt sind und eine Kammer einen proximalen Einlass für ein Kühlmittel und die andere Kammer einen proximalen Auslass für das Kühlmittel hat.

2. Ballonkatheter nach Anspruch 1, **gekennzeichnet durch** einen weiteren Schlauch, der den Mittelschlauch umgibt, sodass ein distal oder proximal offener ringförmiger Raum zwischen dem anderen Schlauch und dem Mittelschlauch entsteht, der das Zurückströmen und den Wiederumlauf des in den Ballon eingeleiteten Druckmediums ermöglicht.

3. Ballonkatheter nach Anspruch 1 oder 2, **dadurch gekennzeichnet, dass** die Heizmittel in dem Ballon angeordnet sind.

4. Ballonkatheter nach Anspruch 1 oder 2, der Umwälzmittel aufweist, die eine Umwälzung des Mediums in dem oder durch den Ballon erzeugen.

5. Ballonkatheter nach Anspruch 2, **dadurch gekennzeichnet, dass** die Heizmittel in dem Zwischenabschnitt angeordnet sind.

6. Ballonkatheter nach Anspruch 2, **dadurch gekennzeichnet, dass** die Heizmittel außerhalb der Abschnitte angeordnet sind.

7. Ballonkatheter nach einem vorhergehenden Anspruch, **dadurch gekennzeichnet, dass** die Heizmittel aus elektrischen Widerstandselementen, PTC-Widerständen, Laserenergie-Emissionsmitteln und externen Wärmetauschern gewählt sind.

8. Ballonkatheter nach einem vorhergehenden Anspruch, **dadurch gekennzeichnet, dass** der axial verlaufende Schlauch zusammen mit dem Zwischenschlauch eine Einheit bildet, die in Bezug auf das distale Ende des Ballons axial verschiebbar ist, und dass das proximale Ende des Ballons an dem distalen Ende der Einheit so befestigt ist, dass die Verstellbarkeit der axialen Länge des Ballons übertragen wird, wobei der Katheter Feststellmittel zum Aufrechterhalten einer gewünschten Ballonlänge aufweist.

9. Ballonkatheter nach einem der Ansprüche 1 bis 7, **dadurch gekennzeichnet, dass** der Mittelschlauch aus mindestens zwei ausziehbaren und konzentrischen, teilweise überlappenden Schläuchen besteht, von denen einer distal herausragt und das distale Ende des Ballons trägt und der andere das proximale Ende des Ballons trägt, wodurch eine relative Axialverschiebung der Schläuche die Ballonlängeneinstellung ermöglicht, wobei der Katheter Feststellmittel zum Aufrechterhalten einer gewünschten Ballonlänge aufweist.

10. Ballonkatheter nach einem vorhergehenden Anspruch, **dadurch gekennzeichnet, dass** die radiale Ausdehnung des axial verlaufenden Schlauchs, des Zwischenschlauchs und des Mittelschlauchs mit den ringförmigen Räumen dazwischen etwa 15 % bis etwa 40 % des Katheterradius einnehmen.

11. Ballonkatheter nach einem vorhergehenden Anspruch, **dadurch gekennzeichnet, dass** der axial verlaufende Schlauch mit axial verlaufenden Innenrippen, die von entsprechenden Außennuten definiert sind, versehen ist, wobei die Rippen die radialen Trennwände bilden, die kurz vor dem distalen Ende des axial verlaufenden Schlauchs enden, wodurch sie die Flüssigkeitskopplung zwischen den Kammern herstellen.

12. Ballonkatheter nach einem der Ansprüche 1 bis 10, **dadurch gekennzeichnet, dass** der Zwischenschlauch mit axial verlaufenden Außennuten versehen ist, die flexible Windungen, die die radialen Trennwände bilden, aufnehmen, wobei die Trennwände kurz vor dem distalen Ende des Zwischenschlauchs enden, wodurch sie die Flüssigkeitskopplung zwischen den Kammern herstellen.

13. Ballonkatheter nach einem vorhergehenden Anspruch zur Behandlung der Prostata, **dadurch gekennzeichnet, dass** der Ballon an seinem proximalen Ende mit einer Wärmedämmung zum Schutz des an den Sphinkter angrenzenden empfindlichen Bereichs versehen ist.

14. Ballonkatheter nach Anspruch 13, **dadurch gekennzeichnet, dass** die Wärmedämmung zum Rektalbereich verläuft, um den Teil der Prostatahöhle zu schützen, der der dünnen Rektumwand gegenüberliegt.

15. Ballonkatheter nach einem vorhergehenden Anspruch, **gekennzeichnet durch** einen radial dehnbaren Stent, der den Ballon umfasst, wobei der Stent so verformbar ist, dass er sich synchron mit der radialen Ausdehnung des Ballons ausdehnt, wenn er in den Gang oder die Höhle eingeführt wird, wodurch er beim Herausziehen des Katheters aus dem Gang oder der Höhle darin gedehnt bleibt.

16. Ballonkatheter nach Anspruch 15, **dadurch gekennzeichnet, dass** der Stent aus einem thermoplastischen, biologisch abbaubaren Polymer besteht, das beim Erwärmen dadurch verformbar ist, dass es einen Erweichungspunkt über der Körpertemperatur hat.

17. Ballonkatheter nach Anspruch 16, **dadurch gekennzeichnet, dass** das Polymer aus PCA, PLLA und PLGA und deren Copolymeren gewählt ist.

18. Ballonkatheter nach einem der Ansprüche 15 bis 17, **dadurch gekennzeichnet, dass** der Stent bei einer Temperatur nahe dem Schmelzpunkt warmgeformt worden ist, um eine gewünschte Stent-Konfiguration bei der Implantation zu erzielen, und dann zu einer gewünschten Konfiguration zum Einführen geformt worden ist.

## Revendications

1. Cathéter à ballonnet pour exercer une pression interne sur un tissu environnant d'un conduit ou d'une cavité de mammifère dans une section de celui-ci, comprenant une section distale allongée et un ballonnet flexible et dilatable accueillant ladite section distale qui, conjointement avec ledit ballonnet, est destinée à être insérée dans ladite section de conduit ou de cavité, comprenant en outre un moyen de fourniture d'un milieu de pression pour dilater ledit ballonnet et un moyen de chauffage pour chauffer ledit milieu, le cathéter comprenant en outre une section intermédiaire et une section proximale pour faire fonctionner le dispositif, lesdites sections distale et intermédiaire contenant un tube central, dont la partie distale est pourvue d'au moins une sortie pour ledit milieu à l'intérieur dudit ballonnet, et dont la partie intermédiaire est entourée d'un tube se prolongeant axialement, à l'extrémité distale duquel l'extrémité proximale dudit ballonnet est attachée, l'extrémité distale dudit ballonnet étant attachée audit tube central, **caractérisé par** un tube intermédiaire positionné de façon concentrique à l'intérieur dudit tube se prolongeant axialement et entourant ledit tube central de manière à former un espace annulaire entre ledit tube central et ledit tube intermédiaire contenant un milieu calorifuge et un second espace annulaire entre ledit tube intermédiaire et ledit tube se prolongeant axialement, le second espace annulaire étant divisé en au moins deux compartiments se prolongeant axialement distincts par au moins deux cloisons radiales opposées, lesdits compartiments étant en communication fluide à leurs extrémités distales, et un compartiment ayant une entrée proximale pour un milieu de refroidissement et un autre compartiment ayant une sortie proximale pour ledit milieu de refroidissement.

2. Cathéter à ballonnet selon la revendication 1, **caractérisé par** un autre tube entourant ledit tube central de manière à former un espace annulaire ouvert distalement et proximalement entre ledit autre tube et ledit tube central, permettant un retour et une recirculation du milieu de pression introduit dans ledit ballonnet.

3. Cathéter à ballonnet selon la revendication 1 ou 2, dans lequel ledit moyen de chauffage est positionné à l'intérieur dudit ballonnet.

4. Cathéter à ballonnet selon la revendication 1 ou 2, comprenant un moyen de circulation générant une circulation dudit milieu à l'intérieur ou à travers ledit ballonnet.

5. Cathéter à ballonnet selon la revendication 2, dans lequel ledit moyen de chauffage est positionné dans ladite section intermédiaire.

6. Cathéter à ballonnet selon la revendication 2, dans lequel ledit moyen de chauffage est positionné à l'extérieur desdites sections.

7. Cathéter à ballonnet selon l'une quelconque des revendications précédentes, dans lequel ledit moyen de chauffage est choisi parmi les éléments de résistance électrique, les résistances CTP, les moyens d'émission d'énergie laser, et les échangeurs de chaleur externes.

8. Cathéter à ballonnet selon l'une quelconque des revendications précédentes, dans lequel le tube se prolongeant axialement, conjointement avec ledit tube intermédiaire, forme une unité qui est mobile axialement par rapport à l'extrémité distale dudit ballonnet, et dans lequel l'extrémité proximale dudit ballonnet est attachée à l'extrémité distale de ladite unité de manière à conférer une capacité de réglage de la longueur axiale du ballonnet, le cathéter comprenant un moyen de blocage pour conserver une longueur de ballonnet souhaitée.

9. Cathéter à ballonnet selon l'une quelconque des revendications 1 à 7, dans lequel ledit tube central est composé d'au moins deux tubes télescopiques et concentriques s'imbriquant partiellement, dont l'un fait saillie distalement et porte l'extrémité distale du ballonnet, et dont l'autre porte l'extrémité proximale du ballonnet, le déplacement axial relatif desdits tubes permettant un réglage de la longueur du ballonnet, le cathéter comprenant un moyen de blocage pour conserver une longueur de ballonnet souhaitée.

10. Cathéter à ballonnet selon l'une quelconque des revendications précédentes, dans lequel le prolongement radial dudit tube se prolongeant axialement, dudit tube intermédiaire et dudit tube central incluant les espaces annulaires entre ceux-ci occupe environ 15% à environ 40% du rayon du cathéter.

11. Cathéter à ballonnet selon l'une quelconque des revendications précédentes, dans lequel ledit tube se prolongeant axialement est pourvu d'arêtes internes se prolongeant axialement définies par des rainures externes correspondantes, lesdites arêtes formant lesdites cloisons radiales s'interrompant avant l'extrémité distale dudit tube se prolongeant axialement, établissant de ce fait ladite communication fluide entre lesdits compartiments.

12. Cathéter à ballonnet selon l'une quelconque des revendications 1 à 10, dans lequel ledit tube intermédiaire est pourvu de rainures externes se prolongeant axialement accueillant des filets flexibles formant lesdites cloisons radiales, lesdites cloisons s'interrompant avant l'extrémité distale dudit tube intermédiaire, établissant de ce fait ladite communication fluide entre lesdits compartiments.

13. Cathéter à ballonnet selon l'une quelconque des revendications précédentes pour le traitement de la prostate, dans lequel ledit ballonnet est pourvu, à son extrémité proximale, d'une isolation thermique pour la protection de la zone sensible proche du sphincter.

14. Cathéter à ballonnet selon la revendication 13, dans lequel ladite isolation thermique est prolongée vers la zone du rectum pour protéger la partie de la cavité prostatique faisant face à la fine paroi rectale.

15. Cathéter à ballonnet selon l'une quelconque des revendications précédentes, **caractérisé par** une endoprothèse dilatable radialement entourant ledit ballonnet, ladite endoprothèse étant déformable pour se dilater de manière synchrone avec la dilatation radiale du ballonnet lorsqu'elle est insérée dans ledit conduit ou ladite cavité, étant de ce fait maintenue dilatée à l'intérieur lors du retrait du cathéter dudit conduit ou de ladite cavité.

16. Cathéter à ballonnet selon la revendication 15, dans lequel ladite endoprothèse est faite d'un polymère thermoplastique biodégradable qui est déformable à la chaleur en ayant un point de ramollissement supérieur à la température corporelle.

17. Cathéter à ballonnet selon la revendication 16, dans lequel ledit polymère est choisi parmi le PCA, le PLLA et le PLGA, et les copolymères de ceux-ci.

18. Cathéter à ballonnet selon l'une quelconque des revendications 15 à 17, dans lequel ladite endoprothèse a été soumise à un formage à chaud à une température proche de la température de fusion pour donner une configuration d'endoprothèse souhaitée lorsqu'elle est implantée, puis façonnée en une configuration souhaitée pour insertion.
